## Europäisches Patentamt
## European Patent Office
## Office européen des brevets

(19)

(11) Numéro de publication: **0 018 872**
**B1**

(12) # FASCICULE DE BREVET EUROPEEN

(45) Date de publication du fascicule du brevet:
**16.10.85**

(21) Numéro de dépôt: **80400488.5**

(22) Date de dépôt: **14.04.80**

(51) Int. Cl.⁴: **A 61 B 6/02,** A 61 B 6/00,
A 61 B 5/04

(54) Procédé de tomodensitométrie et tomodensitomètre adapté à ce procédé.

(30) Priorité: **02.05.79 FR 7911019**

(43) Date de publication de la demande:
**12.11.80 Bulletin 80/23**

(45) Mention de la délivrance du brevet:
**16.10.85 Bulletin 85/42**

(84) Etats contractants désignés:
**DE GB NL**

(56) Documents cités:
**FR - A - 2 237 606**
**FR - A - 2 403 059**
**GB - A - 2 002 987**
**US - A - 4 182 311**

(73) Titulaire: **THOMSON-CSF, 173, Boulevard Haussmann, F-75379 Paris Cedex 08 (FR)**

(72) Inventeur: **Klausz, Rémy, "THOMSON-CSF"-SCPI 173, bld Haussmann, F-75360 Paris Cedex 08 (FR)**

(74) Mandataire: **Chaverneff, Vladimir et al, THOMSON-CSF SCPI 173, Bld Haussmann, F-75379 Paris Cedex 08 (FR)**

ACTORUM AG

## Description

L'invention concerne un procédé de tomodensitométrie et un tomodensitomètre adapté à ce procédé, et particulièrement un procédé de mesure radiodensitométrique ou tomodensitométrique, et un tomodensitomètre, destinés à l'examen de corps animés de mouvements périodiques tels que les tissus cardiaques et péricardiaques, comme décrit dans le document GB-A-2 002 987.

La tomodensitométrie nécessite un grand nombre de mesures effectuées dans des directions variant selon au moins un demi-cercle. Il s'en suit que la durée d'un examen est très longue et peut atteindre plusieurs minutes. Ceci n'a pas d'inconvénient majeur lorsqu'il s'agit d'examiner un organe de faible dimension, tel que le cerveau, et immobile. Il n'en va pas de même lorsque l'on veut examiner le thorax: les mouvements cardiaques (et la respiration) provoquant un flou qui rend l'examen très médiocre, voire inutilisable.

Pour y remédier on a d'abord cherché à diminuer la durée des mesures en utilisant des ensembles munis d'un grand nombre de détecteurs disposés en éventail: on a ainsi réussi à réduire cette durée à quelques secondes. Mais celle-ci est encore beaucoup trop grande pour se situer entre deux périodes, pendant le temps où les mouvements produits par les battements du cœur sont faibles. C'est pourquoi on a proposé d'effectuer les mesures sur une fraction quelconque du cycle cardiaque, au cours de nombreux cycles consécutifs. Toutes les mesures sont faites sur cette même fraction, et on obtient ainsi un effet stroboscopique donnant une image figée du tissu à l'instant choisi du cycle de mouvement. Cet instant est autant que possible choisi au début de la période de relative immobilité du cœur (diastole) séparant deux périodes de contraction (systoles).

Si l'on obtient ainsi des résultats améliorés pour l'examen du cœur, il n'en va pas de même pour l'examen de tissus péricardiaques. En effet la durée des mesures est très augmentée par rapport à ce qu'elle était lorsque ces mesures étaient effectuées d'une manière continue, puisqu'elles ont lieu dans ce cas par petites séries au cours d'un grand nombre de fractions de cycles. Pendant la durée de ces mesures, d'autres mouvements interviennent, produits notamment par la respiration ou les simples déplacements du patient. Par ailleurs l'application de ce procédé entraîne une complication de l'appareillage. Enfin ce procédé est limité à l'examen de tissus animés de mouvements cycliques.

L'objet de l'invention est un procédé de tomodensitométrie d'un corps dont une partie mobile est animée de périodes de mouvement séparées par des périodes d'immobilité, lesdits mouvements se faisant essentiellement suivant une direction moyenne prédéterminée, du type consistant à faire tourner autour dudit corps un ensemble de mesure comprenant une source de rayons pénétrants, émettant un faisceau en direction de détecteurs et à recueillir pour certaines positions angulaires dudit ensemble de mesure un nombre de mesures suffisant pour permettre une reconstruction d'image, caractérisé en ce qu'il consiste à déterminer les mouvements cycliques de ladite partie mobile et à commander l'acquisition dudit nombre de mesures suffisant pour permettre une reconstruction en fonction desdits mouvements de façon à faire coïncider les mesures prises pendant une période de mouvement précitée avec des positions angulaires dudit ensemble de mesures pour lesquelles la direction moyenne dudit faisceau est sensiblement parallèle à ladite direction moyenne des mouvements de la partie mobile précitée.

Lorsque le nombre de ces directions de déplacement linéaire est insuffisant, le procédé n'est applicable que si les périodes de mouvements sont suivies de périodes d'immobilité.

Dans ce cas les mesures sont poursuivies, pendant la période d'immobilité qui suit la période de mouvement, dans les directions suivant lesquelles la projection des mouvements, s'ils avaient lieu, n'aurait jamais été nulle.

Lorsque les mouvements des parties mobiles sont cycliques, et sont répartis dans le temps en périodes de mouvement suivies de périodes d'immobilité, à une fréquence sensiblement fixe, les mesures commencent pendant une période d'immobilité, et sont poursuivies pendant la période de mouvement et la période d'immobilité suivante, les mesures effectuées pendant la période de mouvement s'effectuant dans une direction moyenne suivant laquelle la projection du mouvement est sensiblement nulle, c'est-à-dire dans une direction sensiblement parallèle à celle du mouvement.

L'invention sera mieux comprise et d'autres caractéristiques apparaîtront au cours des explications et de la description d'une réalisation particulière, données ci-après avec les figures qui représentent:

la fig. 1, un ensemble de graphiques relatifs à des mouvements cycliques et à leurs effets au cours des mesures, représentés en fonction du temps, et

la fig. 2, le schéma d'un tomodensitomètre selon l'invention.

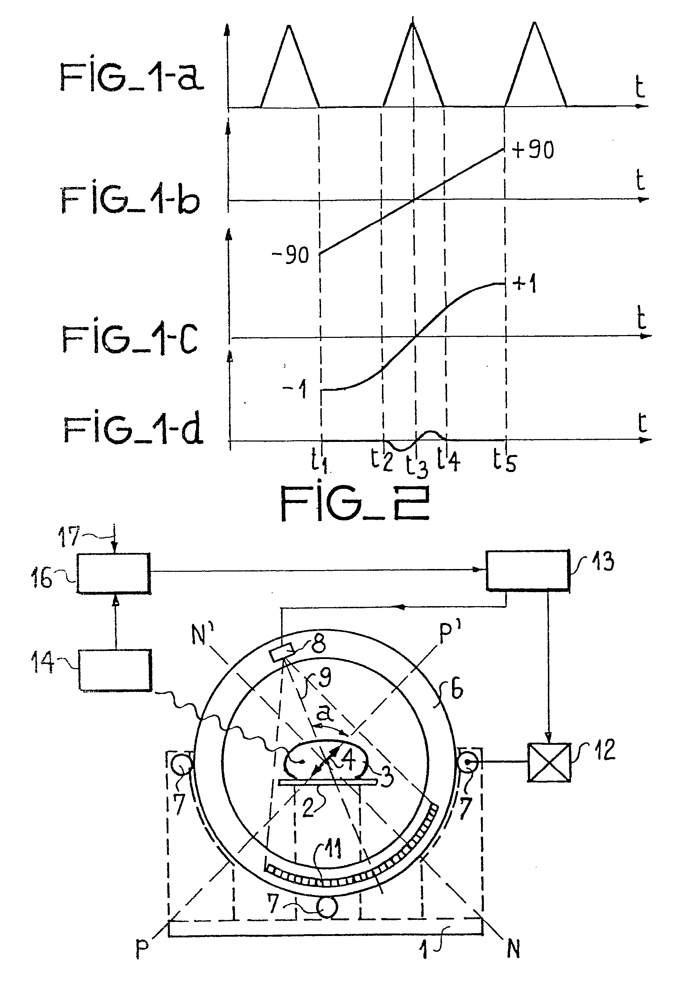

Les courbes de la fig. 1 sont relatives aux mouvements cardiaques ou des tissus péricardiaques. On peut considérer que ces mouvements sont assimilables à des déplacements sensiblement linéaires selon une direction connue, en négligeant les mouvements de déformation ou de rotation.

Sur la courbe de la fig. 1a on a représenté en ordonnée l'amplitude du mouvement du tissu à examiner. On voit qu'un cycle se compose d'une période d'immobilité de $t_1$ à $t_2$, puis d'une période de mouvement $t_2$ à $t_4$ avec un point culminant en $t_3$. Dans le cas de mouvements cardiaques les périodes d'immobilité $t_1$ $t_2$, $t_4$ $t_5$ d'une part et la période de mouvement $t_2$ $t_4$ d'autre part durent environ 0,4 seconde chacune.

Selon l'invention, on fait commencer les mesures à l'instant $t_1$ et on s'arrange pour qu'au point de départ la direction moyenne des rayons émis par la source fasse un angle de 90 degrés avec la direction du mouvement. La durée de rotation de l'ensemble de mesure a été réglée pour que, à l'instant $t_5$ il se trouve à son point d'arrivée. Pour ce dernier également, l'angle que forme la direction moyenne des rayons émis par la source avec la direction du mouvement fait un angle de 90 degrés, l'angle décrit par l'ensemble de mesure entre la position de départ et la position d'arrivée étant de 180 degrés environ.

On a tracé sur la courbe de la fig. 1b l'angle que fait la direction moyenne des rayons avec la direction du mouvement; cette courbe est une droite variant de $-90$ à $+90$ degrés par rapport à cette direction. On remarquera qu'à l'instant $t_3$, où la direction moyenne des rayons est parallèle à la direction du mouvement, ce dernier est à sont amplitude maximum (fig. 1a).

La courbe de la fig. 1c représente en ordonnée la valeur du sinus des angles représentés en ordonnée de la courbe de la fig. 1b.

L'effet du mouvement sur les mesures est proportionnel au produit de l'amplitude du mouvement (fig. 1a) par le sinus de l'angle fait par la direction moyenne des rayons avec la direction du mouvement (fig. 1c). Les valeurs de cet effet sont tracées sur la fig. 1d. On voit que de $t_1$ à $t_2$ et de $t_4$ à $t_5$ le mouvement étant nul, son effet est nul; de $t_2$ à $t_3$ et de $t_3$ à $t_4$ l'effet n'est pas nul mais il est faible en raison de la faible valeur du sinus.

Au total en appliquant le procédé de l'invention, en effectuant les mesures entre le début d'une période d'immobilité et la fin de la période d'immobilité suivante, et en s'arrangeant pour que la direction moyenne des rayons soit sensiblement parallèle à la direction du mouvement à l'instant où le mouvement est à son maximum d'amplitude, l'effet du mouvement sur les mesures est très faible. De plus la durée totale des mesures est courte environ 1,5 seconde puisqu'elles sont effectuées sans interruption du début à la fin, même pendant la période de mouvement. Cette courte durée permet d'éviter les mouvements produits par d'autres causes que les battements de cœur, par exemple la respiration ou tout autre déplacement du corps à examiner.

La fig. 2 donne le schéma d'un appareil adapté à ce procédé. Un socle 1 supporte une table d'examen 2 sur laquelle est placé le corps à examiner 3 dont la zone à examiner est animée de mouvements élémentaires de déplacements cycliques le long d'un segment 4, dont l'orientation PP' est située sensiblement dans le plan d'examen et supposée connue. Un ensemble de mesure, représenté par une couronne 6 roulant sur des galets 7 solidaires du socle 1, comporte une source 8 qui émet un faisceau de rayons X en forme d'éventail, dont la direction moyenne représentée par la ligne pointillée 9 fait avec la ligne PP' un angle a, en direction d'un ensemble de détecteurs de mesure 11.

L'orientation de l'ensemble de mesure, autour d'un axe sensiblement parallèle au plan de la table d'examen 2, est commandée par un moteur 12 lui-même commandé par un circuit de commande 13. Un appareil de détection 14 des mouvements cycliques, qui peut être un électro-cardiographe, est relié au corps à examiner 3. Il envoie à un module de synchronisation 16 des signaux concernant les mouvements cycliques dudit corps. Le module 16 reçoit en 17 les signaux concernant le début des mesures en provenance d'un opérateur et il transmet au circuit de commande 13, en fonction des signaux qu'il a reçus de l'appareil de détection 14 et de l'opérateur 17, les signaux nécessaires à l'initiation et au déroulement des mesures et commande ainsi d'une part l'émission de la source 8 et d'autre part la mise en route du moteur 12.

Le fonctionnement de l'appareil est le suivant:

Le module de synchronisation 16 reçoit de l'appareil de détection 14 des signaux analoques à la forme de la courbe de la fig. 1a donnant le début et la fin de chaque période de mouvement et d'immobilité. Il élabore et envoie au circuit de commande 13 un signal tel que l'ensemble de mesure effectue une rotation de l'ordre de 180 degrés pendant le temps $t_5-t_1$.

Avant d'initier les mesures, l'opérateur affiche une position de départ des mesures en N correspondant à une position où la direction moyenne des rayons 9 fait avec le segment 4 un angle de 90 degrés.

Lorsque l'opérateur envoie en 17 le signal de départ des mesures, le module 16 ne le transmet pas directement au circuit 13, mais il le retarde et ne l'envoie que quand l'appareil de détection 14 aura signalé le début $t_1$ d'une période d'immobilité. Le circuit 13 transmet alors à la source le signal d'émission, et au moteur un signal de mise en route tel que l'ensemble de mesure parvient à sa position de départ en N à l'instant $t_1$ et à sa position d'arrivée en N' à l'instant $t_5$ qui est la fin de la période d'immobilité suivante.

Il est bien entendu que l'invention n'est pas limitée à l'exemple de réalisation particulier qui vient d'être décrit. Par exemple il n'est pas nécessaire d'utiliser, pour effectuer les mesures, la totalité des périodes d'immobilité précédant et suivant le mouvement, ni de choisir comme position de départ des mesures celle dans laquelle la direction moyenne des rayons est perpendiculaire à la direction du mouvement. On peut commencer et terminer les mesures à des instants quelconques du cycle, à condition que, lorsque le mouvement se produit, la direction moyenne des rayons soit sensiblement confondue avec celle du mouvement.

D'autre part les applications du procédé selon l'invention concernent l'examen d'autres tissus que les tissus cardiaques ou péricardiaques et même d'autres examens que médicaux par exemple l'examen de corps dont les mouvements peuvent être décomposés en une série de trajets linéaires.

L'invention est également applicable aux tomodensitomètres dans lesquels l'ensemble de mesure comporte plusieurs sources de rayons X réparties sur un cercle ou une portion de cercle autour de l'objet examiné de façon à limiter, voir supprimer le mouvement de rotation de l'ensemble source-détecteurs. La suppression du mouvement de rotation est particulièrement avantageuse dans le cas où l'objet, ou une partie de l'objet, est animé de mouvements successifs sensiblement linéaires dans un grand nombre de directions différentes. Le module de synchronisation et le circuit de commande sont alors réalisés de façon que lorsqu'un mouvement est détecté le tube qui se trouve sensiblement dans le prolongement de la direction du mouvement émette un faisceau de rayons X vers l'objet.

On peut également utiliser pour supprimer le mouvement de rotation de l'ensemble sources-détecteurs et éviter l'emploi de plusieurs tubes à rayons X, un tube unique dans lequel l'anode de forme circulaire ou semi-circulaire qui entoure l'objet examiné est balayée par un ou plusieurs faisceaux d'électrons émis par une ou plusieurs cathodes.

## Revendications

1. Procédé de tomodensitométrie d'un corps (3) dont une partie mobile (4) est animée de périodes de mouvement séparées par des périodes d'immobilité, lesdits mouvements se faisant essentiellement suivant une direction moyenne prédéterminée (PP'), du type consistant à faire tourner autour dudit corps un ensemble de mesure comprenant une source de rayons pénétrants, émettant un faisceau en direction de détecteurs et à recueillir pour certaines positions angulaires dudit ensemble de mesure un nombre de mesures suffisant pour permettre une reconstruction d'image, caractérisé en ce qu'il consiste à déterminer les mouvements cycliques de ladite partie mobile et à commander l'acquisition dudit nombre de mesures suffisant pour permettre une reconstruction en fonction desdits mouvements de façon à faire coïncider les mesures prises pendant une période de mouvement (t2, t4) précitée avec des positions angulaires dudit ensemble de mesures pour lesquelles la direction moyenne dudit faisceau est sensiblement parallèle à ladite direction moyenne des mouvements de la partie mobile précitée.

2. Tomodensitomètre comportant un support (2) sur lequel est placé le corps (3) à examiner, dont une partie mobile (4) est animée de périodes de mouvement séparées par des périodes d'immobilité, lesdits mouvements se faisant essentiellement suivant une direction moyenne (PP') prédéterminée, comportant en outre un ensemble de mesure comprenant une source de rayons pénétrants émettant un faisceau en direction de détecteurs (11) et susceptible d'effectuer des mesures sur au moins 180° autour du corps à examiner et un circuit de commande (13) dudit ensemble de mesure, commandant la prise des mesures et les directions moyennes correspondantes dans lesquelles s'effectuent lesdites mesures, caractérisé en ce qu'il comporte en outre un appareil de détection (14) des mouvements de ladite partie mobile et un module de synchronisation (16) recevant dudit appareil de détection des informations concernant lesdites périodes de mouvement et lesdites périodes d'immobilité et comportant des moyens pour faire varier le début de l'actionnement dudit circuit de commande de manière que la direction moyenne du faisceau devienne sensiblement parallèle à la direction moyenne prédéterminée (PP') précitée pendant une période de mouvement de ladite partie mobile, ledit module de synchronisation délivrant le signal de début de prises de mesures au cours de la période d'immobilité précédente.

## Claims

1. Method of tomodensitometric examination of a body (3) at least one movable part (4) of which performs periods of movement separated by periods without movement, said movements being essentially performed along a predetermined mean direction (PP'), of the type consisting in rotating about said body a measurement set comprising a source of penetrating radiation emitting a beam in the direction of detectors and collecting for determined angular positions of said measurement set a sufficient number of measurements to permit an image reconstruction, characterized in that it consists in determining the cyclic movements of said movable part and in controlling the acquisition of said number of measurements sufficient to permit a reconstruction as a function of said movements in a manner to cause the measurements taken during one of said periods of movements (t2, t4) to be coincident with the angular positions of said measurement set for which the mean direction of said beam is substantially parallel to said mean direction of the movements of said movable part.

2. Tomodensitometric apparatus comprising a carrier (2) wherein the body (3) to be examined is placed and a movable part (4) of which performs periods of movement separated by periods without movement, said movements being essentially performed along a predetermined mean direction (PP'), further comprising a measurement set comprising a source of penetrating radiation emitting a beam in the direction of detectors (11), and susceptible to perform measurements about at least 180° about the body to be examined, and a circuit (13) for controlling said measurement set, controlling the taking of the measurements and the corresponding mean directions in which said measurements are performed, characterized in that it further comprises a detection apparatus (14) for detecting the movements of said movable part and a synchronization module (16) receiving from said detection apparatus information concerning said periods of movement and said periods without movement, and comprising means for causing the beginning of the actuation of said control circuit to vary in such a manner that the mean direction of the beam is caused to be substantially parallel to the predetermined mean direction (PP') during a period of movement of said movable part, said synchronization module supplying the signal of the beginning of the measurement taking in the course of the preceding period without movement.

## Patentansprüche

1. Verfahren zur tomodensitometrischen Untersuchung eines Körpers (3), wovon ein beweglicher Teil (4) Bewegungsperioden ausführt, welche durch Perioden ohne Bewegung getrennt sind, wobei die genannten Bewegungen im wesentlichen entlang einer vorbestimmten mittleren Richtung (PP') erfolgen, vom Typ, der darin besteht, dass um den genannten Körper herum eine Messgruppe verdreht wird, die eine Quelle durchdringender Strahlung umfasst, die ein Bündel in Richtung von Detektoren aussendet, und dass für bestimmte Winkelstellungen der genannten Messgruppe eine Anzahl von Messwerten aufgenommen wird, die ausreicht, um eine Bildrekonstruktion zu gestatten, dadurch gekennzeichnet, dass es darin besteht, die zyklischen Bewegungen des genannten beweglichen Teiles zu bestimmen und die Erfassung der genannten Anzahl von zur Bildrekonstruktion ausreichenden Messwerten in Abhängigkeit von den genannten Bewegungen derart zu steuern, dass die während einer der genannten Bewegungsperioden (t2, t4) aufgenommenen Messwerte zusammenfallen mit Winkelstellungen der Messgruppe, für welche die mittlere Richtung des genannten Bündels im wesentlichen parallel zu der genannten mittleren Richtung der Bewegungen des genannten beweglichen Teiles ist.

2. Tomodensitometer mit einem Träger (2), auf welchem der zu untersuchende Körper (3) angeordnet ist, wovon ein beweglicher Teil (4) Bewegungsperioden ausführt, die durch Perioden ohne Bewegung getrennt sind, wobei die genannten Bewegungen im wesentlichen entlang einer vorbestimmten mittleren Richtung (PP') erfolgen, ferner mit einer Messgruppe, die eine Quelle durchdringender Strahlung umfasst, welche ein Bündel in Richtung von Detektoren (11) aussendet, und welche geeignet ist, Messungen über wenigstens 180° um den zu untersuchenden Körper herum auszuführen, und mit einer Steuerschaltung (13) zur Steuerung der Messgruppe, welche die Aufnahme von Messwerten und die mittleren entsprechenden Richtungen steuert, in welchen die genannten Messungen durchgeführt werden, dadurch gekennzeichnet, dass es ferner ein Detektionsgerät (14) zur Erfassung der Bewegungen des genannten beweglichen Teiles und ein Synchronisationsmodul (16) enthält, welcher von dem Detektionsgerät Informationen empfängt, welche die genannten Bewegungsperioden sowie die genannten Perioden ohne Bewegung betreffen, und ferner Mittel enthält, um den Beginn des Wirksamwerdens der genannten Steuerschaltung derart zu verändern, dass die mittlere Richtung des Bündels im wesentlichen parallel zur vorbestimmten mittleren Richtung (PP') während einer Bewegungsperiode des genannten beweglichen Teiles wird, wobei das Synchronisationsmodul das Signal für den Beginn der Aufnahme von Messwerten im Verlaufe der vorausgehenden bewegungsfreien Periode abgibt.

FIG_1-a

FIG_1-b

+90

−90

FIG_1-C

+1

−1

FIG_1-d

$t_1$  $t_2$  $t_3$  $t_4$  $t_5$

FIG_2